Europäisches Patentamt

European Patent Office

Office européen des brevets

(19)

(11) **EP 1 464 336 A1**

(12) **EUROPEAN PATENT APPLICATION**

(43) Date of publication:
**06.10.2004 Bulletin 2004/41**

(51) Int Cl.7: **A61K 31/4985**, A61P 43/00,
G01N 33/50

(21) Application number: **03007683.0**

(22) Date of filing: **03.04.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(71) Applicants:
• **Humboldt-Universität zu Berlin
D-10099 Berlin (DE)**
• **Universitätsklinikum Charité der Humboldt-
Universität zu Berlin
10098 Berlin (DE)**

(72) Inventors:
• **Herrmann, Andreas, Prof. Dr.
13156 Berlin (DE)**
• **Lage, Hermann, Dr.
13353 Berlin (DE)**
• **Woehlecke, Holger, Dr.
10179 Berlin (DE)**

(74) Representative: **Krauss, Jan
Forrester & Boehmert,
Pettenkoferstrasse 20-22
80336 München (DE)**

(54) **Use of tryprostatin A for the reversal of cancer drug resistance**

(57)     The invention relates to novel uses of tryprostatin A.

The phenomenon of multidrug resistance (MDR) in human cancers is one of the major causes of failure of chemotherapy. The fungal secondary metabolitc try-prostatin A (TPS-A), a dike-topiperazine, has been shown to overcome multi-drug resistance, and to inhibit lipid transport, whilst no cytotoxicity of the compound itself could be observed.

EP 1 464 336 A1

## Description

The invention relates to novel uses of tryprostatin A.

Resistance to therapy with anticancer drugs is a major clinical problem often resulting in treatment failure. Recently, a substantial amount of investigations have been performed, aimed at the circumvention or reversal of drug resistance.

Various members of the protein superfamily of ATP-binding cassette transporters (ABC-transporters) have been associated with multidrug resistance (MDR) of human cancers when overexpressed (overview in Gottesman MM et al., Nat Rev Cancer 2002;2:48-58). Clinical MDR of malignancies to many antineoplastic agents, the major obstacle in the successful treatment of cancer, may be in part due to increased activity of these broad specificity plasma membrane localized drug efflux pumps, like the most extensively studied MDR transporter P-glycoprotein (P-gp) (overview in Ling V. Cancer Chemother Pharmacol 1997;40(Suppl):S3-S8). The energy-dependent xenobiotics transport activity results in decreased intracellular concentrations of antitumor agents. Meanwhile, P-gp-depending MDRs are often designated as classical MDR.

A relatively new member of the ABC-transporter family is the "breast cancer resistance protein" (BCRP, MXR, ABCG2) (overview in Lage H et al., Lancet Oncol 2000;1:169-75). BCRP is a 72 kDa ABC-"half-transporter" consisting of 655 amino acid residues, which is thought to homo- or heterodimerize to form an active transport complex. The transporter BCRP is overexpressed in a variety of multidrug-resistant human cancer cell lines, exhibiting an atypical MDR or non-Pgp-mediated MDR phenotype. Elevated expression of BCRP results in resistance of various cancer cell lines to antitumor drugs including mitoxantrone, topotecan, daunorubicin, doxorubicin or bisantrene (Ross DD et al., J Natl Cancer Inst 1999;91:429-33). Transfection experiments using the BCRP encoding cDNA also confer an atypical multidrug-resistant phenotype to former drug-sensitive cancer cells (Doyle LA et al. Proc Natl Acad Sci USA 1998;95: 15665-70). High expression levels of BCRP were also detected in blast cells from patients with acute leukemia, whose malignant disease was drug-resistant (Ross DD et al., Blood 2000;96:365-8; Sargent JM et al., Br J Haematol 2001; 115:257-62). Moreover, BCRP has been identified and characterized as a novel stem cell transporter (Zhou S et al., Nat Med 2001;7:1028-34). In a murine system, it could be demonstrated that BCRP expression was highly conserved in primitive stem cells from a variety of sources including hematopoietic and embryonic stem cells. The level of BCRP protein expression directly correlated with the stem cell-characterizing side population phenotype.

Inhibitors of ABC-transporters are potential agents of clinical interest for resensitization of certain drug-resistant tumor cells by maintaining lethal intracellular concentrations of anticancer drugs. Furthermore, inhibition of these cell surface pump molecules present at different tissue barriers may open ways to alter the pharmacokinetics of certain drugs. Multidrug-resistant phenotypes were found to be circumvented by a variety of pharmacological products, designated as MDR modulators or chemosensitizers, whereby some of them have already been tested in the clinic. However, up to now only a few substances were identified to be able to reverse multidrug-resistance: the *Aspergillus fumigatus* secondary metabolite fumitremorgin C (FTC) (Rabindran SK et al., Cancer Res 2000;60:47-50), its derivative demethoxy-FTC (van Loevezijn A et al., Bioorg Med Chem Lett 2001;11:29-32), the so-called second-generation MDR modulator GF120918 (de Bruin M et al., Cancer Lett 1999;146:117-26), the quinazoline-based HER family tyrosine kinase inhibitor CI1033 (Erlichman C et al., Cancer Res 2001;61;739-48), experimental camptothecin analogues (Maliepaard M et al., Clin Cancer Res 2001;7:935-41; Perego P et al., Cancer Res 2001;61:6034-37), and estrogens like estrone and 17β-estradiol (Imai Y et al., Jpn J Cancer Res 2002;93:231-5). In addition the ribozyme technology has been applied successfully to a certain extent to reverse a BCRP-mediated drug-resistant phenotype (Kowalski P et al., Cancer Gene Ther 2001;8:185-92; Kowalski P et al., Cancer Gene Ther 2002;9:579-86).

None of the above-identified compounds have proven particularly useful in clinical applications, and some of them, like, for example, fumitremorgin C are cytotoxic themselves and can only be applied in concentrations in the range of from 1 μM to approximately 10 μM.

To provide some further background to the present invention, typically, lipids of the plasma membrane of mammalian cells are distributed asymmetrically between both membrane halves: sphingomyelin (SM) is restricted to the outer, exoplasmic, leaflet and phosphatidylserine (PS) to the inner, cytoplasmic, leaflet in virtually all eukaryotic cells studied, while phosphatidylcholine (PC) and phosphatidylethanolamine (PE) appear to show some preference for the outer (PC) respectively the inner (PE) leaflet (Zachowski, A. (1993) *Biochem. J.* **294**, 1-14).

This asymmetric lipid arrangement plays a role in a number of cellular processes. Several studies have shown that its regulation may be important for membrane budding and fusion during vesicle trafficking (for review see (Devaux, P. (2000) *Biochimie* **82**, 497-509)). Alterations of this non-random lipid distribution may have pivotal physiological consequences, such as the removal of apoptotic cells by macrophages upon surface exposure of PS (Zwaal, R., and Schroit, A. (1997) *Blood* **89**, 1121-1132).

Although passive movement of lipids across a membrane is generally slow, this process tends to abolish lipid asymmetry in membranes. Therefore, the non-random transbilayer distribution of lipids requires maintenance and regulation by membrane proteins specifically and coordinately controlling lipid movement across the bilayer.

Indeed, the aminophospholipids PS and PE are rapidly transported from the exoplasmic to the cytoplasmic leaflet by an active ATP-dependent and protein-mediated process maintaining lipid asymmetry (Devaux, P. (2000) *Biochimie* **82**, 497-509; Seigneuret, M. and Devaux, P. F. (1984) *Proc.Natl.Acad.Sci. USA* **81**, 3751-3755; Daleke, D. L., and Lyles, J.V. (2000) *Biochim. Biophys. Acta* **1486**, 108-127).The responsible protein, the so-called aminophospholipid translocase, is very likely a member of the P-type ATPase family. In contrast, the movement of PC and SM from the outer to the inner plasma membrane leaflet is typically slow and not mediated by transport proteins.

Over the last couple of years, various lipids and lipid analogues were shown to be transported by members of the ATP-binding cassette (ABC) transporter superfamily: The ABCB family member MDR3 Pgp (ABCB4) and its mouse homologue mdr2 Pgp, specifically expel PC from the cell (Smit, J., Schinkel, A., Oude Elferink, R., Groen, A., Wagenaar, E., van Deemter, L., Mol, C., Ottenhoff,R., van der Lugt, N., van Roon, M., van der Valk, M., Offerhaus, G., Berns, A., and Borst, P. (1993) *Cell* **75**, 451-462;

Ruetz, S., and Gros, P. (1994) *Cell* **77**, 1071-1081; Smith, A., Timmermans-Hereijgers, J., Roelofsen, B., Wirtz, K., van Blitterswijk, W., Smit, J., Schinkel, A., Borst, P. (1994) *FEBS Lett* **354**, 263-266; van Helvoort, A., Smith, A., Sprong, H., Fritzsche, I., Schinkel, A., Borst, P., and van Meer, G. (1996) *Cell* **87**, 507-517.), while MDR1 Pgp (ABCB1), involved in multidrug resistance (MDR) of tumor cells (Cardarelli, C., Aksentijevich, I., Pastan, I., and Gottesman, M. (1995) *Cancer Res.* **55**, 1086-1091), transports various lipid analogues (van Helvoort, A., Smith, A., Sprong, H., Fritzsche, I., Schinkel, A., Borst, P., and van Meer, G. (1996) *Cell* **87**, 507-517; Bosch, I., Dunussi-Joannopoulos, K., Wu, R., Furlong, S., and Croop, J. (1997) *Biochemistry* **36**, 5685-5694; Abulrob, A., and Gumbleton, M. (1999) *Biochem. Biophys. Res. Commun.* **262**, 121-126; Pohl, A., Lage, H., Müller, P., Pomorksi, T., and Herrmann. A. (2002) *Biochem. J.* **365,** 259-268) and probably also the endogenous lipids PAF (platelet activating factor, a short-chain PC), PS, and glucosylceramide (Pohl, A., Lage, H., Müller, P., Pomorksi, T., and Herrmann. A. (2002) *Biochem. J.* **365**, 259-268; Ernest, S., and Bello-Reuss, E. (1999) *J. Am. Soc. Nephrol.* **10**, 2306-2313; Raggers, R. J., Pomorski, T., Holthuis, J. C. M., Kalin, N., and van Meer, G. (2000) *Traffic* **1**, 226-234) from the inner to the outer plasma membrane leaflet. MRP1 (ABCC1), belonging to the ABCC family, was reported to mediate outward transport of C6-NBD-PC and -PS in murine erythrocytes (16,17) and of NBD-sphingolipids in epithelial cells (Raggers, R., van Helvoort, A., Evers, R., and van Meer, G. (1999) *J. Cell Sci.* **112**, 415-422), while the ABCA family member ABCA1 was shown to transport PS in various mammalian cells (Marguet, D., Luciani, M. F., Moynault, A., Williamson, P., and Chimini, G. (1999) *Nature Cell Biol.* **1**, 454-456; Hamon, Y., Broccardo, C., Chambenoit, O., Luciani, M-F., Toti, F., Chaslin, S., Freyssinet, J.-M., Devaux, P. F., Neish, J., Marguet, D., and Chimini, G. (2000) *Nature Cell Biol.* **2**, 399-406).

The potential lipid transporters MDR1 Pgp, MDR3 Pgp, MRP1 and ABCA1 each contain two nucleotide binding domains and two transmembrane domains (three in MRP1). Half-size ABC transporters containing only one nucleotide binding domain and one transmembrane domain have recently been implicated with the transport of lipids as well, for example White (ABCG1), an ABCG subfamily member which is involved in the regulation of cholesterol and phospholipid transport in macrophages and possibly in other cells, as it is ubiquitously expressed in human tissues (Klucken, J., Büchler, C., Orso, E., Kaminski, W. E., Porsch-Özcürümez, M.., Liebisch, G., Kapinsky, M., Diederich, W., Drobnik, W., Dean, M., Allikmets, R., and Schmitz, G. (2000) *Proc. Natl. Acad. Sci USA .* **97**, 817-822).

The recently discovered human half-size transporter Breast Cancer Resistance Protein (BCRP, MXR, ABCG2), also a member of the ABCG family, is a 72 kDa Protein consisting of 655 amino acids (overview in (Lage, H., and Dietel, M. (2000) *Lancet Oncol.* **1**, 169-175)). It is thought to homo- or heterodimerize to form an active transport complex. The *BCRP* gene is highly overexpressed in the plasma membrane of several drug-resistant cell lines where it has been shown to confer atypical multi drug resistance (MDR) (Ross, D.D., Yang, W., Abruzzo, L.V., Dalton, W.S., Schneider, E., Lage, H., Dietel, M., Greenberger. L., Cole, S.P., and Doyle, L.A. (1999) *J. Natl. Cancer Inst.* **91**, 429-433; . Doyle, L.A, Yang, W., Abruzzo L.V., Krogmann T., Gao, Y., Rishi, A.K., and Ross, D.D. (1998) *Proc Natl Acad Sci USA* **95**, 15665-15670; Litman, T., Brangi, M., Hudson, E., Fetsch, P., Abati, A., Ross, D.D., Miyake, K., Resau, J.H., and Bates, S.E. (2000) *J.Cell. Science* **113**, 2011-2021; Rocchi, E., Khodjakov, A. Volk, E.L., Yang, C.H., Litman, T., Bates, S.E., and Schneider, E. (2000) *Biochem Biophys Res Commun.* **271**, 42-46). BCRP transports the antitumor drugs mitoxantrone, topotecan, daunorubicin, doxorubicin and bisantrene (Ross, D.D., Yang, W., Abruzzo, L.V., Dalton, W.S., Schneider, E., Lage, H., Dietel, M., Greenberger. L., Cole, S.P., and Doyle, L.A. (1999) *J. Natl. Cancer Inst.* **91**, 429-433).

In the *MDR1* overexpressing human gastric carcinoma cell line EPG85-257RDB, the present inventors have previously demonstrated MDR1 Pgp mediated outward transport of the PS analogue C6-NBD-PS and of endogenous PS (Pohl, A., Lage, H., Müller, P., Pomorksi, T., and Herrmann. A. (2002) *Biochem. J.* **365,** 259-268). No such lipid transport has yet been determined for BCRP, nor have there been many compounds identified that interfere with the action of BCRP, which compounds are particularly useful in clinical, pharmaceutical and/or pharmacological applications.

Accordingly, there exists a need in the art for new compounds and new ways to reverse multidrug-resistance. It has therefore been a object of the present invention to provide for a new way of reversal of multidrug-resistance. In particular, it has been an object of the present invention to provide for a new way of reversal of BCRP-mediated forms of multidrug resistance. It has also been an object of the present invention to provide for a new way of reversal of multidrug-resistance, in particular atypical multidrug resistance, wherein the agent responsible for the reversal is non-cytotoxic even at high concentrations. It has furthermore been an object of the present invention to provide for reversal of multidrug-resistance wherein there are fewer adverse side-effects.

It has also been an object of the present invention to provide for novel methods of interfering with the action of BCRP. It has also been an object of the present invention to provide for compounds, which compounds act on BCRP.

All these objects are solved by the use of tryprostatin A for the manufacture of a medicament for the treatment of a cancerous disease, wherein a reversal of drug resistance in cancer cells is desired.

The objects of the present invention are also solved by the use of tryprostatin A for the identification of compounds that reverse a BCRP-mediated drug resistance.

The objects of the present invention are furthermore solved by a method of screening for compounds capable of chemosensitizing multidrug-resistant cells comprising the steps:

- Administering a subject compound to be tested to a non P-gp/non MRP multiple drug resistant cell,
- administering an anti-cancer pharmaceutical to which the cell is resistant,
- measuring a response, and
- comparing the response with a response achieved by administering tryprostatin A to a non P-gp/non MRP multiple drug resistant cancer cell of the same type, that has not been treated with the subject compound to be tested.

Also, the objects of the present invention are solved by a compound identified by the method according to the present invention, said compound having a DMF $\geq$ 2, said DMF being defined by the formula

$$\text{DMF} = \frac{\text{IC}_{50} \text{ (in the absence of tryprostatin)}}{\text{IC}_{50} \text{ (in the presence of tryprostatin)}} \text{ ,}$$

$\text{IC}_{50}$ being the concentration of an anti-cancer pharmaceutical at which said anti-cancer pharmaceutical achieves half-maximum inhibition of growth of cancer cells.

and said compound causing an increase of drug accumulation of $\geq$ 40%, wherein said increase of drug accumulation is the ratio of drug accumulation measured in the presence and absence of tryprostatin A

In one aspect of the use according to the present invention, the drug resistance is a multidrug resistance.

Preferably, a dose modifying factor (DMF) is $\geq$ 1, more preferably 2, even more preferably 6, the DMF being defined by the formula

$$\text{DMF} = \frac{\text{IC}_{50} \text{ (in the absence of tryprostatin A)}}{\text{IC}_{50} \text{ (in the presence of tryprostatin A)}}$$

$\text{IC}_{50}$ being the concentration of an anti-cancer pharmaceutical at which said anti-cancer pharmaceutical achieves half-maximum inhibition of growth of cancer cells.

In a preferred embodiment, the DMF 60, wherein, more preferably, the concentration of tryprostatin A is 5 $\mu$M, preferably in the range 5 - 20 $\mu$M, even more preferably approximately 10 $\mu$M.

In one aspect of the use according to the present invention, the drug resistance is a resistance against a compound/combination of compounds selected from the group comprising mitoxantrone, camptothecin-analogues, in particular topotecan, doxorubicin, bisantrene, vinblastin, paclitaxel and colchicin.

Preferably, the drug resistance is a non P-gp-mediated/non MRP drug resistance, wherein, more preferably, the drug resistance is a BCRP-mediated drug resistance.

In one embodiment, the cancerous disease is selected form the group comprising breast, gastric, brain, prostate, liver, colon, ovarian, testicular, small intestinal cancer, non-Hodgkin's lymphoma, and leukemia.

In one embodiment, an increase of drug accumulation of $\geq$ 40% in the cancer cells is desired, said increase of drug accumulation being a ratio of drug accumulation measured in the presence and absence of tryprostatin A.

Preferably, tryprostatin A is administered concomitantly with an anti-cancer pharmaceutical, wherein, more preferably, the anti-cancer pharmaceutical is selected from the group comprising mitoxantrone, camptothecin-analogues, in particular topotecan, doxorubicin, bisantrene, vinblastin, paclitaxel and colchicin.

In one embodiment, tryprostatin A is used in a concentration in the range of from 1 $\mu$M - 100 $\mu$M, preferably 1 $\mu$M - 80 $\mu$M, more preferably 1 $\mu$M - 50 $\mu$M.

In one embodiment the medicament for the treatment of a cancerous disease furthermore contains a pharmaceutically acceptable carrier and/or diluent.

Preferably, the cancerous disease is in a human.

Also the objects of the present invention are solved by the use of tryprostatin A for the manufacture of a medicament for the treatment of diseases characterised by or associated with a lipid transport in and out of cells. Preferably, the lipid transport in and out of cells is BCRP-mediated. Preferably, the disease characterised by a lipid transport, more preferably by a BCRP-mediated lipid transport, is a cancerous disease.

The objects of the present invention are also solved by the use of tryprostatin A or any protonated form thereof as a pharmacological tool.

Preferably the pharmacological tool is a tool wherein interference with and/or inhibition of BCRP is desired.

Furthermore, the objects of the present invention are solved by the use of tryprostatin A or any protonated form thereof for inhibiting lipid transport, preferably BCRP-mediated lipid transport.

As used herein, the term "reversal of drug resistance in cancer cells" is meant to designate any process, whereby a cancer cell becomes sensitive to an anti-cancer-drug, such that this drug may exert an inhibitory effect on the proliferation of the cell, again. The term is not restricted to any particular mechanism.

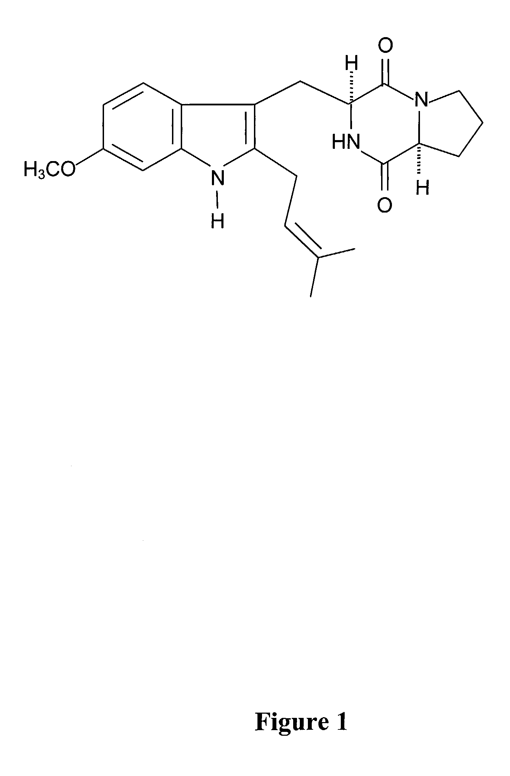

The term "tryprostatin A" is meant to designate any compound having the formula as shown in figure 1, and any protonated form thereof.

The term "cancerous disease" is meant to signify any disease characterized by a malignant proliferative behavior of cells which do not obey the growth behavior normally encountered for such cells within a healthy organism.

The term "dose modifying factor" is as defined above and represents a ratio of the $IC_{50}$ values obtained in the absence and the presence of tryprostatin A. It represents a measure for the ability of tryprostatin A to sensitize a cancerous cell towards the action of an anti-cancer-drug.

The term "non P-gp/non MRP drug resistance" is meant to signify a drug resistance that cannot be attributed to the presence or expression of the ABC transporter P-glycoprotein (P-gp), a transmembrane protein member, nor to the presence or expression of the ABC-transporter multi-drug resistance protein (MRP 1).

The term "BCRP-mediated drug resistance" is meant to signify a drug resistance characterized by the presence or expression of the breast cancer resistance protein (BCRP), which is also sometimes referred to as the mitoxantrone resistance gene (MXR) or placenta-specific ABC transporter (ABCP).

The term "lipid transport" is meant to signify any translocation of lipids in and out of cells. It further encompasses a movement of lipids within a lipid membrane, with the lipid that is being transported, not necessarily exiting from or leaving the membrane.

The inventors have surprisingly found that tryprostatin A can be used as a reversing agent for multidrug-resistance, in particular a BCRP-mediated drug-resistance. Advantageously, and in contrast to other known reversing agents, tryprostatin A can be used in concentrations 10 μM, without showing any cytotoxic side effects in different cancer cell cultures. This makes tryprostatin A a prime candidate for clinical applications and as a laboratory tool for screening experiments. It can also be used as an excellent pharmacological tool or in a pharmacological assay, wherein an inhibitor of BCRP is needed.

Tryprostatin A (TPS-A) (Fig. 1) is an *Aspergillus fumigatus* secondary metabolite and belongs to the substance class of diketopiperazines. TPS-A was purified originally from the culture broth of *Aspergillus fumigatus* BM939 during the screening of natural products that interfere with cell cycle progression (Cui CB et al., J Antibiot 1995;48:1382-4; Cui CB et al., J Antibiot 1996;49:527-33; Cui CB et al., J Antibiot 1996;49:534-40). This compound arrested cell cycle progression at the $G_2$/M phase at final concentrations of 125 μM. Moreover, it was reported that TPS-A is an inhibitor of microtubule-associated protein (MAP)-dependent microtubule assembly and, through the disruption of the microtubule spindle, specifically inhibits cell cycle progression at the M phase at a final concentration of 250 μM TPS-A (Usui T et al., Biochem J 1998;333:543-8).

Reference is now made to the figures wherein,

Figure 1 shows the structure of TPS-A.

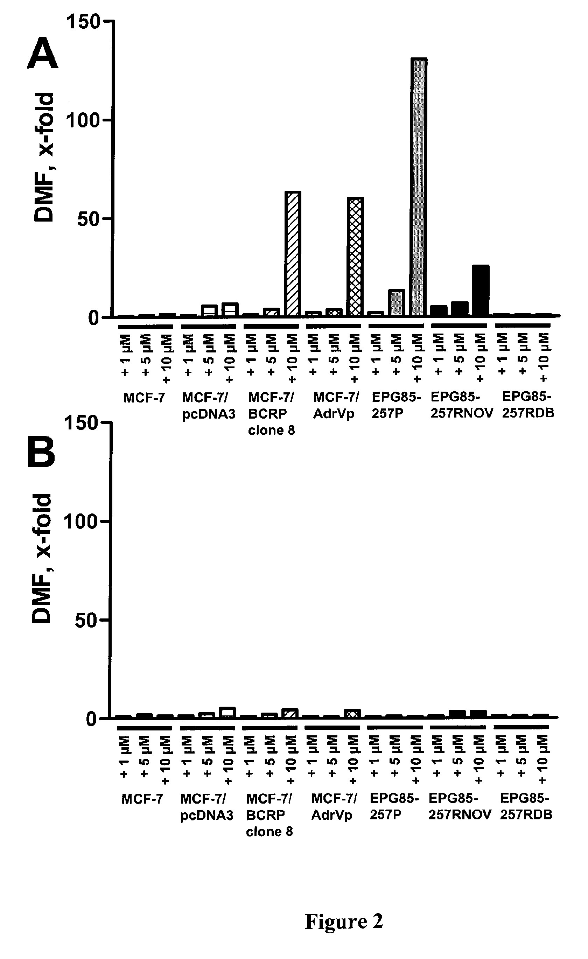

Figure 2 shows the effect on the dose modifying factor (DMF) of TPS-A on cytotoxicity of (A) mitoxantrone and (B) daunorubicin in drug-sensitive and drug-resistant gastric and breast carcinoma cells.

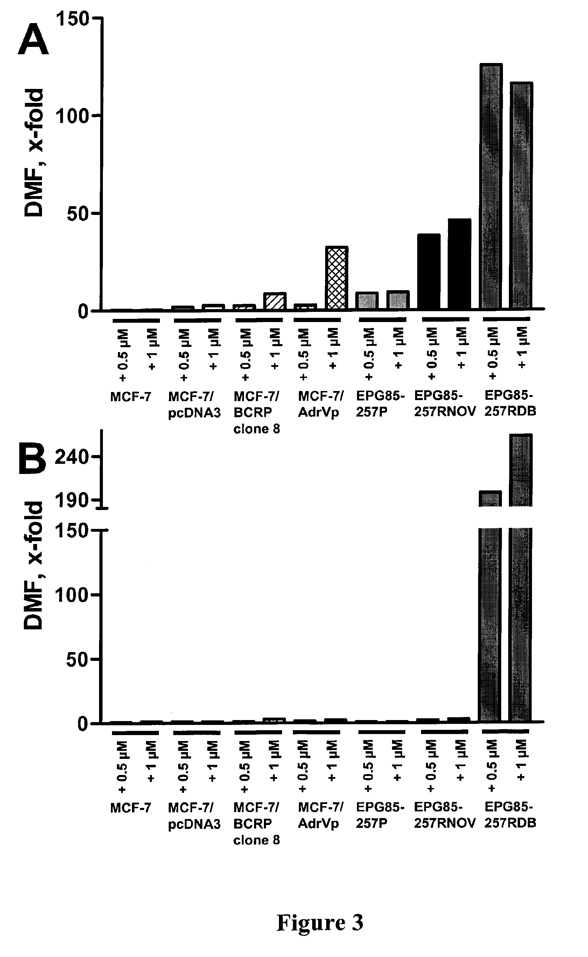

Figure 3 shows the effect on the dose modifying factor (DMF) of GF 120918 on cytotoxicity of (A) mitoxantrone and (B) daunorubicin in drug-sensitive and drug-resistant gastric and breast carcinoma cells.

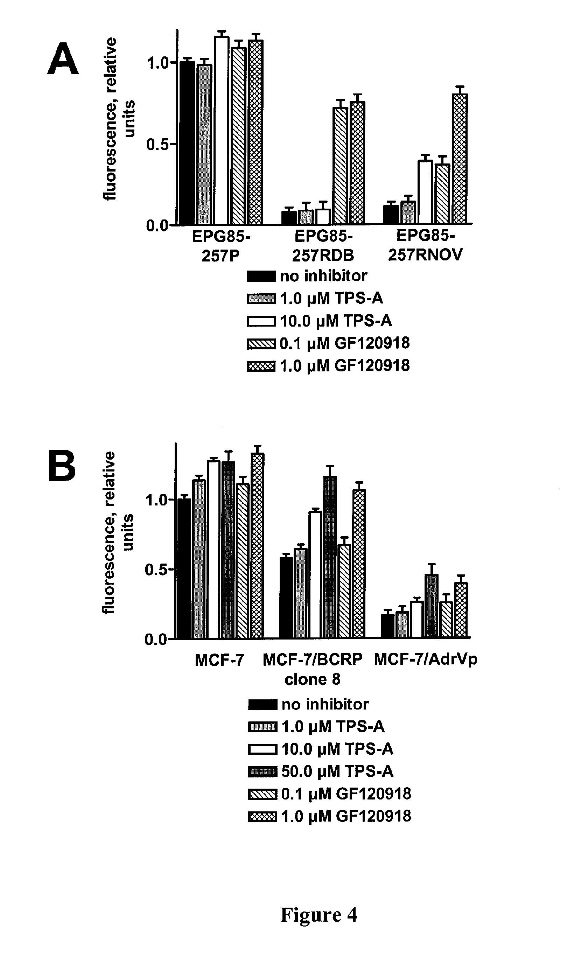

Figure 4 shows the mitoxantrone accumulation in drug-sensitive and drug-resistant human carcinoma cells. Cells were exposed to 5 μM mitoxantrone for 60 min at 37 °C with increasing ABC-transporter inhibitor concentrations of TPS-A or GF120918. Fluorescence intensity was measured by flow cytometry. In each case the mean value was normalized to the parental, non-resistant carcinoma cell line without any inhibitor. Error bars represent SD of at least three triplicate determinations. (A) Gastric carcinoma cells; (B) breast cancer cells.

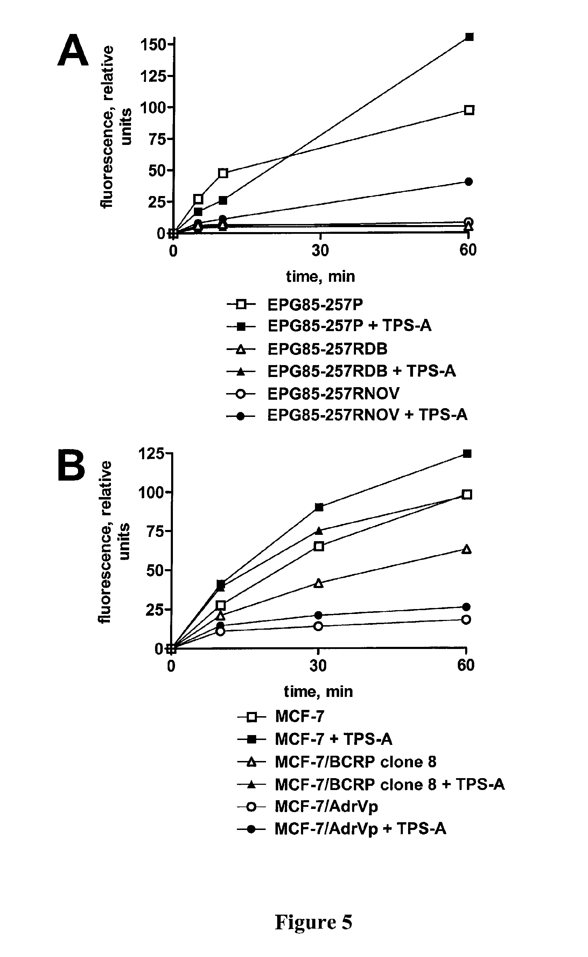

Figure 5 shows the time dependence of mitoxantrone accumulation in drug-sensitive and drug-resistant human carcinoma cells. Cells were incubated with 10µg/ml mitoxantrone for increasing periods at 37°C with or without 10 µM TPS-A or 10 µM GF 120918, respectively. In each case the mean values were normalized to the parental, non-resistant carcinoma cell line without any inhibitor. (A) Gastric carcinoma cells; (B) breast cancer cells.

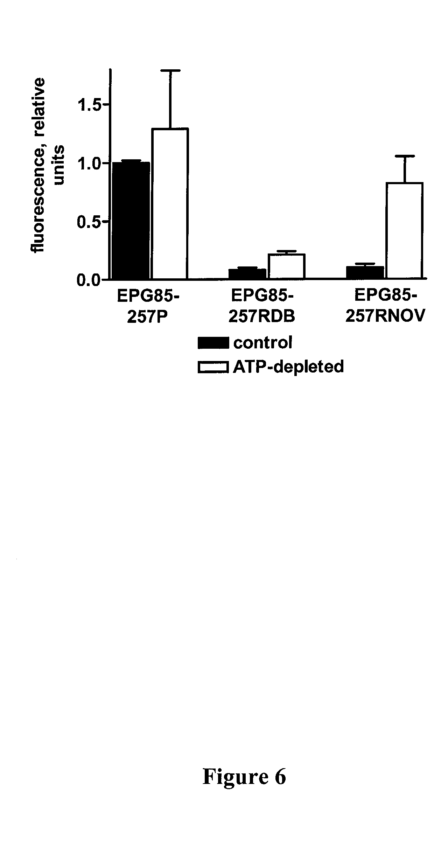

Figure 6 shows the mitoxantrone accumulation under energy-deprived conditions in drug-sensitive, classical MDR, and atypical MDR gastric carcinoma cells. After 120 min preincubatation under ATP-depleting conditions cells were exposed to 10 µM mitoxantrone for 60 min at 37°C. Fluorescence intensity was measured by flow cytometry. The mean value was normalized to the parental, non-resistant gastric carcinoma cell line EPG85-257P grown under normal conditions. Error bars represent SD of at least three triplicate determinations.

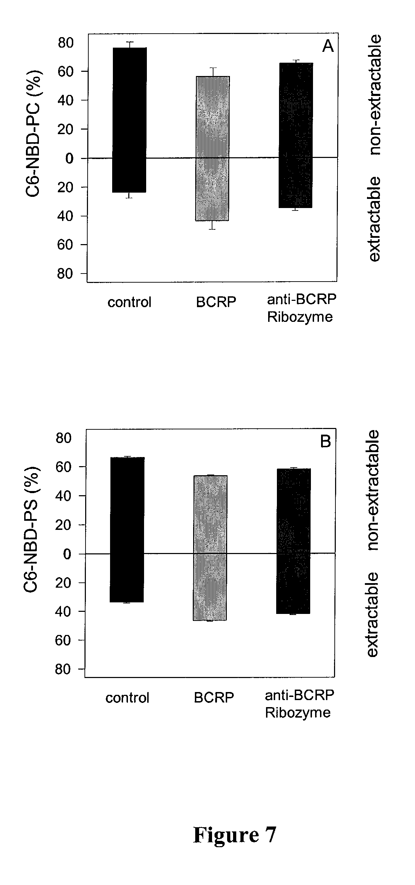

Figure 7 shows the outward translocation of C6-NBD-PC (A) and C6-NBD-PS (B) in *BCRP* overexpressing gastric carcinoma cells (EPG85-257RNOV), *anti-BCRP*-ribozym transfected (EPG85-257RNOV-RzB1) and control cells (EPG85-257P). A. Cells were incubated with 25 µM C6-NBD-PA for 180 minutes at 15°C. To trap the fluorescent lipid products (C6-NBD-PC) appearing on the cell surface in the extracellular medium, incubation was performed in the presence of 1% BSA. B. Cells were labelled with 5 µM C6-NBD-PS and incubated at 20°C for 30 minutes to allow intracellular accumulation of the NBD analogue. C6-NBD-PS remaining on the cell surface was then extracted twice by incubation with 2% (w/v) BSA in mPBS for 10 minutes on ice. Then, cells were incubated at 37°C for 30 minutes. Fluorescent lipids (C6-NBD-PS and metabolites) appearing on the cell surface were extracted and quantified as described in Materials and Methods. Data represent the means ± S.E.M. of at least n = 2 independent experiments in duplicate.

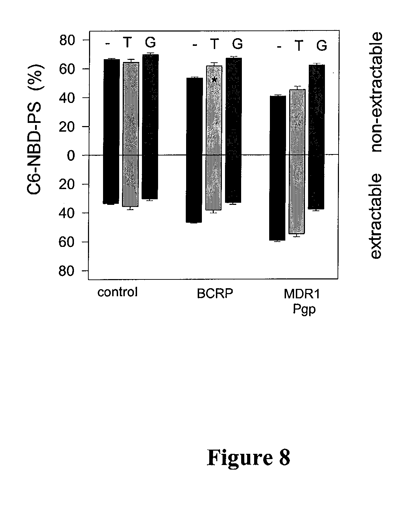

Figure 8 shows the inhibition of the outward translocation of C6-NBD-PS in *BCRP* overexpressing cells, *MDR1 Pgp* overexpressing cells (EPG85-257RDB) and control cells at 37°C by Tryprostatin A and GF120918. Cells were preincubated for 10 minutes on ice in the absence of BCRP inhibitors (-) and in the presence of 10 µM Tryprostatin A (T) or GF120918 (G), labelled with 5 µM C6-NBD-PS and incubated at 20°C for 30 minutes to allow intracellular accumulation of the NBD analogue. C6-NBD-PS remaining on the cell surface was then extracted twice by incubation with 2% (w/v) BSA in mPBS for 10 minutes on ice. Then, cells were incubated at 37°C for 30 minutes. Fluorescent lipids (C6-NBD-PS and metabolites) appearing on the cell surface were extracted and determined as described in Materials and Methods. Data represent the means ± S.E.M. of at least n = 2 independent experiments in duplicate. Data were analyzed by a two-way analysis of variance Tukey test (Jandel SigmaStat 2.0) considering treatment and day of experiment. The asterisks denote a significant difference compared to the respective cells without inhibitor pretreatment (p<0.05).

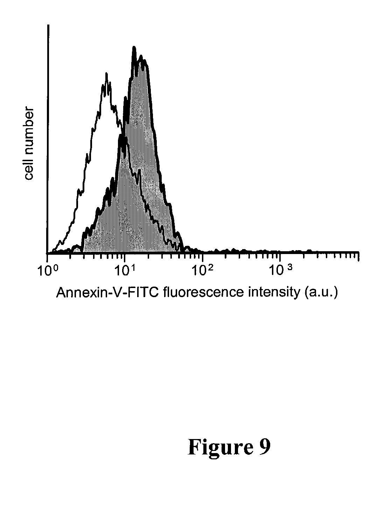

Figure 9 shows a flow cytometric analysis of PS localisation in the outer membrane leaflet by FITC-Annexin V binding to *BCRP* overexpressing cells (EPG85-257RNOV). Histograms show fluorescence intensity due to FITC-Annexin binding at the surface of *BCRP* overexpressing cells in the absence (grey filled graph) and in the presence of 5µM of Tryprostatin A (white filled graph). The histogram of control cells (EPG85-257P) was similar to that of *BCRP* overexpressing cells I the presence of Tryprostatin A (not shown).

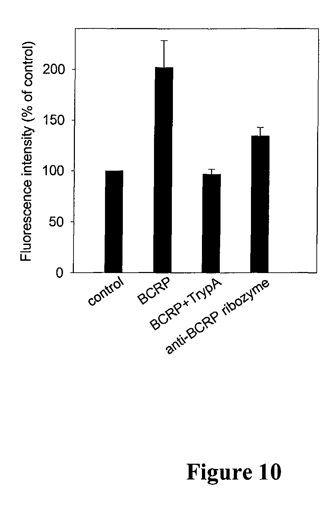

Figure 10 shows a flow cytometric analysis of PS localisation in outer membrane leaflet by FITC-Annexin V binding to *BCRP* overexpressing cells, *anti-BCRP*-ribozyme-transfected cells, and control cells. Annexin binding to *BCRP* overexpressing cells was reduced to the level of control cells after preincubation with 5 µM Tryprostatin A for 30 min at 37°C. Cells were colabelled with the membrane-impermeable nucleic acid stain propidium iodide as described in Materials and Methods. Cells showing elevated propidium iodide staining were excluded. 10000 cells were counted per sample. Data are the mean ± S.E.M. of n=3 or n=2 (anti-BCRP-ribozyme-transfected cells) independent experiments of duplicate or triplicate measurements. For each experiment, the control was set to 100%.

The invention will now be more fully understood by the following specific examples which are offered for purposes of illustration, not limitation of the invention.

### Example 1

**Chemicals, cell lines and culture conditions**

TPS-A was purified from the culture broth of *Aspergillus fumigatus* BM939 (FERM P-15067), which was originally isolated from a sea sediment sample collected from the sea bed (760 m deep) at the mouth of the Oi river, Shizuoka prefecture, Japan, as described in Cui CB et al., J Antibiot 1996;49:527-33. GF120918 was kindly provided by GlaxoWellcome (Stevenage, U.K.). As antineoplastic agents mitoxantrone (Lederle, Wolfratshausen, Germany), daunorubicin and doxorubicin (Farmitalia Carlo Erba, Freiburg, Germany) were used. G418 was obtained from Invitrogen (San Diego, CA, USA) and verapamil was purchased from Sigma (Munich, Germany).

The human gastric carcinoma cell line EPG85-257P and its drug-resistant sublines were established in the inventors' laboratory described in Dietel M et al., Cancer Res 1990;50:6100-6; Lage H et al., Int J Hyperthermia 2000; 16:291-303. The cell line EPG85-257RDB shows a classical P-gp-dependent MDR phenotype that was established by exposure to daunorubicin in vitro. The cell variant EPG85-257RNOV exhibits an atypical MDR phenotype accompanied by enhanced expression of BCRP (Ross DD et al., J Natl Cancer Inst 1999;91:429-33) that was established by treatment with mitoxantrone in vitro. The human breast cancer cell lines MCF-7, its atypical multidrug-resistant, BCRP overexpressing derivative MCF-7/AdrVp, the BCRP cDNA-transfected subline MCF-7/BCRP clone 8, and the control clone MCF-7/pcDNA3, transfected with an empty expression vector (Doyle LA et al. Proc Natl Acad Sci USA 1998;95:15665-70) were kindly provided by Douglas D. Ross (Greenebaum Cancer Center of the University of Maryland, Baltimore, MD, USA). These carcinoma-derived cell lines were grown in Leibovitz L 15 medium (Bio Whittaker, Grand Island, NY, USA), 1 mM L-glutamine, 6.25 mg/ml fetuin, 80 IE/l insulin, 2.5 mg/ml transferrin, 1 g/l glucose, 1.1 g/l $NaHCO_3$, 1% minimal essential vitamins, and 20,000 kIE/l trasylol in a humidified atmosphere of 5% $CO_2$ at 37 °C. Additionally, EPG85-257RDB cells were grown with 2.5 µg/ml daunorubicin and the atypical MDR cell line EPG85-257RNOV was grown in the presence of 0.2 µg/ml mitoxantrone. The drug-resistant MCF-7/AdrVp cell line was continuously maintained in the presence of 1 µg/ml doxorubicin and 2.5 µg/ml verapamil. In addition, the medium used for cultivation of transfected MCF-7 sublines contained 400 µg/ml G418.

## Example 2

### Cytotoxicity Assay for Cell Survival

Modulation of chemoresistance was tested using a proliferation assay based on sulforhodamine B (SRB), a protein-binding reagent (Skehan P et al., J Natl Cancer Inst 1990;82:1107-12), as described in Lage H et al., Int J Hyperthermia 2000;16:291-303; Lage H et al., FEBS Lett 2001;503:179-84; Lage H et al., FEBS Lett 2001;494:54-9. In each experiment, 350 cells per well were seeded in the presence or absence of TPS-A in 96-well plates in complete medium and allowed to attach overnight. Mitoxantrone or daunorubicin were added in a dilution series in triplicate wells. After 5 days, when unselected wells were still subconfluent, incubation was terminated by replacing the medium with 10 % trichloroacetic acid, followed by incubation at 4 °C for 1 h. After samples were washed 5 times with water and air-dried, 50 µl 0.4 % SRB (Sigma, St. Louis, MO) in 1 % acetic acid was added and samples were incubated for 10 min at room temperature. Unbound dye was eliminated by washing the plates 5 times with 1 % acetic acid. After air-drying and re-solubilisation of the protein-bound dye in 10 mM Tris-HCl (pH 8.0), absorbance was quantified at 562.5 nm. To determine the $IC_{50}$-value, the absorbance difference of control cells without drug was set to be 100 %. Linear regression of data was done for the linear part of the curve, and $IC_{50}$-values were calculated from multiple, at least three independent experiments for each cell line.

The cytotoxicity of the MDR modulators TPS-A and GF120918 on drug-sensitive and drug-resistant gastric and breast cancer cell lines was initially assessed. No evidence of cross-resistance could be detected in any of the multidrug-resistant tumor cell lines. At concentrations of 10 µM TPS-A and 1 µM GF120918 no growth inhibitory effect could be observed in any of the drug-sensitive or drug-resistant cancer cell lines tested. In MCF-7 breast cancer-derived cell variants even 50 µM TPS-A could be added to the cell culture medium without any effect on the cell proliferation, showing that TPS-A is not cytotoxic.

The cellular sensitivity to mitoxantrone and daunorubicin was assessed by determining the $IC_{50}$ values without or with increasing concentrations of TPS-A or GF120918 as a positive control in the different cancer cell variants. Table 1 presents the data of TPS-A reversal of mitoxantrone and daunorubicin resistance in different gastric and breast carcinoma cell variants, respectively. As control, Table 2 shows the reversal of mitoxantrone and daunorubicin resistance using GF120918 as MDR-reversing agent in the panel of gastric and breast cancer cell lines. In each case, the equation

$$DMF = \frac{IC_{50}(-CS)}{IC_{50}(+CS)}$$

was used to calculate the dose modifying factor (DMF), comparing the $IC_{50}$ values in the presence (+CS) or absence (-CS) of the corresponding chemosensitizer (CS) TPS-A or GF120918.

As shown in Table 1 and Figure 2A, application of TPS-A results in a dose-depending sensitization of BCRP-expressing gastric and breast cancer cells to treatment with mitoxantrone.

No significant effect on sensitivity to daunorubicin could be observed in any of the drug-sensitive, or P-gp-, or BCRP-expressing carcinoma cells by exposure to increasing concentrations to TPS-A (Table 1; Figure 2B).

By adding the MDR modulator GF120918, a dose-depending resensitization to mitoxantrone of BCRP- and P-gp-expressing stomach and mamma carcinoma-derived cell lines could be achieved (Table 2; Figure 3A). In the case of cross-resistance against daunorubicin, a considerable dose-depending sensitization of classical MDR, P-gp-expressing gastric carcinoma EPG85-257RDB cells could be demonstrated, whereas no significant effect could be observed in the other human gastric and breast cancer cell lines (Table 2; Figure 3B).

## Example 3

### Mitoxantrone Accumulation Assay

Cellular mitoxantrone accumulation was determined according to the procedure described in Lage H et al., FEBS Lett 2001;503:179-84. Briefly, $4 \times 10^5$ cells were seeded out in 6-well plates 78 hours prior to the experiment. Incubation with mitoxantrone (0, 1,5 and 10 μg/ml) was performed for 60 minutes at 37°C and 70-80 % cellular confluence. For kinetics of intracellular enrichment of mitoxantrone, cells were exposed to 10 μg/ml mitoxantrone for 0, 5, 10 and 60 min. Cells were washed with ice-cold PBS, trypsinized and resuspended in 4 °C PBS at a concentration of $4 \times 10^5$ cells/ml. Intracellular fluorescence of mitoxantrone was determined by flow cytometry (Becton-Dickinson, San Jose, CA). The cells were excited at 480 nm and emission was measured at 630 nm. A minimum of $10^4$ cells was analyzed for each sample. All assays were performed at least in three independent experiments, each time with triplicate wells.

In order to investigate accumulation under energy-deprived conditions, 120 min prior to loading the cells with mitoxantrone, the medium was changed to glucose-free, pyruvate-free DMEM (GibcoBRL) containing 5 % FCS, plus 10 mM sodium azide to inhibit oxidative phosphorylation, as described in Stein U et al., Int J Cancer 2002; 97: 751-60. Afterwards, cells were exposed to 10 μM mitoxantrone for 60 min at 37 °C and incubated in drug-free medium as described above, but under engergy-deprived conditions.

To provide confirmation that the observed resensitization to mitoxantrone of BCRP-overexpressing cells was indeed the result of BCRP inhibition by TPS-A, a mitoxantrone accumulation assay was performed. The known so-called "multispecific" MDR modulator GF120918, inhibiting P-gp- and BCRP activity, was used as a positive control for P-gp- and BCRP antagonism.

As shown in Fig 4A, TPS-A inhibited in a dosage-depending manner the mitoxantrone pump activity. At concentrations of 10 μM, in the BCRP-positive, atypical MDR gastric carcinoma cell line EPG85-257RNOV, an increase to 40 % of the mitoxantrone accumulation level of untreated drug-sensitive cells was observed. No significant drug transporter inhibiting effect could be demonstrated in the P-gp-expressing, classical MDR variant EPG85-257RDB. A teeny TPS-A effect, leading to a slightly increased mitoxantrone accumulation could be found in the parental, drug-sensitive gastric carcinoma cell variant EPG85-257P. This effect might be explained by a TPS-A-mediated inhibition of the weak constitutive BCRP expression in the parental cell line.

Fig 4 also demonstrates a dosage-depending ABC-transporter inhibition by the control chemosensitizer GF120918. 1 μM of GF12918 showed an 80 % mitoxantrone accumulation inhibiting effect in the BCRP-overexpressing cell line EPG85-257RNOV, as well as in the P-gp-positive EPG85-257RDB cell line an inhibition of mitoxantrone pump activity by 75 %. Likewise, a very weak transporter inhibiting effect could be observed in the parental cell variant.

Similar to the gastric carcinoma system, ABC-transporter activity modulating effects by TPS-A and GF120918 were found for the panel of breast cancer-derived cell lines (Fig 4B). For both MDR modulators, TPS-A and GF120918, a dosage-depending effect could be observed. 50 μM TPS-A or 1 μM GF120918 were sufficient to inhibit the mitoxantrone pump activity completely in the BCRP-transfected cell variant MCF-7/BCRP clone 8, when compared to the drug accumulation level in the untreated, parental control cell line.

The used chemosensitizer concentrations were not sufficient to increase the mitoxantrone accumulation rate for the extremely high resistant cell variant MCF-7/AdrVp, exhibiting an acquired drug-resistance phenotype accompanied by a tremendous high BCRP expression level. 50 μM TPS-A revealed an increase of drug accumulation of 45 % with respect to control cells whereas 40 % were obtained by using 1 μM GF 120918.

In the drug-sensitive, parental cell line MCF-7, only a weak dosage-depending effect of TPS-A and GF120918 on drug accumulation could be observed.

To analyze the time course of MDR modulating effects of TPS-A, drug-sensitive, and drug-resistant gastric carcinoma and breast cancer cells were incubated with 10 μM mitoxantrone and TPS-A for increasing time periods. As shown in Fig 5A and 5B, for both cell systems the chemosensitizing effects of TPS-A could be observed in a time-dependent manner.

Because energy-deprived conditions affect the ATP supply for ABC-transporters like P-gp and BCRP, the impact of these conditions on mitoxantrone accumulation was examined. For this approach, drug-sensitive and drug-resistant gastric carcinoma cells were incubated with glucose-, and pyruvate-free cell culture medium containing sodium azide to inhibit oxidative phosphorylation. As shown in Fig 6, ATP-depletion caused a considerable increase in drug accumulation in the BCRP-overexpressing, atypical MDR cell line EPG85-257RNOV by more than 80 % of the respective accumulation level in the control cells under non-energy-deprived conditions. Only a weak effect of energy deprivation could be detected in the classical MDR variant as well as in the parental, drug-sensitive cells. However, the small effects of ATP-depletion in these cells could be explained by the inhibition of the weak constitutive expressed BCRP transporter.

## Example 4

GF120918 had been demonstrated before to be a more potent inhibitor of P-gp than inhibitors, such as verapamil and cyclosporin A, and was capable of restoring drug sensitivity at concentrations as low as 0.1 $\mu$M (Hyafil F et al., Cancer Res 1993;53:4595-602). From the previous examples, however, it has become clear that, for reverting BCRP-mediated or P-gp-mediated mitoxantrone resistance in the human gastric carcinoma cell line EPG85-257RNOV and EPG85-257RDB, respectively, 1 $\mu$M GF120918 was not sufficient to restore drug sensitivity completely. The same was true in the case of the BCRP-overexpressing breast cancer variants MCF7/BCRP clone 8 and MCF-7/AdrVp. In this case, TPS-A was more ineffective than GF120918 meaning that higher concentrations of TPS-A were necessary than of GF 120918. In the MCF-7-derived breast cancer model system 50 $\mu$M TPS-A were necessary to obtain a level of drug sensitivity beyond that found for 1 $\mu$M GF120918. However, importantly, TPS-A did not show any cytotoxic or growth-inhibiting effects at those dosages. This observation is in line with investigations on the cytotoxicity of TPS-A reported previously. At final concentrations of 125 $\mu$M TPS-A was demonstrated to arrest cell cycle progression at the $G_2$/M phase checkpoint, at 250 $\mu$M TPS-A is an inhibitor of MAP-dependent microtubule assembly (Usui T et al., Biochem J 1998;333:543-8). However, in spite of these cytotoxic effects at very high concentrations TPS-A does not show any cytotoxicity in the concentration range 1-100 $\mu$M and therefore is useful as an MDR modulator, as shown above.

MDR modulating effects of TPS-A and GF120918 were tested in cancer cells exhibiting a very high level of drug resistance in vitro. In the clinical situation a 2-fold or 3-fold increase of drug resistance could prevent the success of a chemotherapeutic treatment. Most likely, in human tumors the degree of resistance mediated by BCRP may be much lower. Thus, the level of pharmacological potency TPS-A needs to have an MDR-reversing effect on cancer cells expressing BCRP may be considerably less in the in vivo situation.

One may wonder whether TPS-A inhibits the pump activity of BCRP, by direct interaction with this ABC-transporter molecule. Without wishing to be bound by any theory, the inventors presently believe that a direct interaction by TPS-A appears most probable because TPS-A is a planar, multi-ring structure like the anticancer drugs mitoxantrone, doxorubicin, or camptothecin-analogues, such as topotecan. Due to its chemical structure, TPS-A may compete with these anticancer compounds for the binding sites on the transport molecule. This mechanism would be similar to alternative ABC-transporters, such as P-gp, for which it had been demonstrated that P-gp substrate molecules and P-gp-inhibiting agents directly interact with the transporter molecule (Loo TW et al., Biochim Biophys Acta 1999;1461: 315-25).

In conclusion, TPS-A reverses multidrug-resistance, in particular BCRP-mediated multidrug-resistance, and has no or very little cytotoxicity which makes it very useful as an MDR modulator in clinical applications.

## Example 5

## Materials

Diisopropyl fluorophosphate (DFP) and fatty acid(FA)-free BSA were purchased from Sigma-Aldrich (Steinheim, Germany), Dulbecco's phosphate buffered saline with $Ca^{2+}$, $Mg^{2+}$(PBS) were obtained from Biochrom KG (Berlin, Germany), PBS was supplemented with 24 mM glucose and 10 mM Hepes (mPBS) = modified Dulbecco's phosphate buffered saline. C6-NBD-phosphatidic acid (C6-NBD-PA), and -PS were purchased from Avanti Polar Lipids (Birmingham, AL, USA), high performance thin layer chromatography plates from Merck (Darmstadt, Germany). FITC-Annexin V, binding buffer and propidium iodide were bought from VPS diagnostics (Hoeven, The Netherlands). Inhibitors of ABC transporters used: Tryprostatin A (gift from H.Osada, Riken-Institute, Japan), GF120918 (gift from Glaxxo Wellcome, UK). Tryprostatin A and GF120918 stocks were prepared in methanol. For all experiments, the effect of the solvent was determined.

**Cell lines and culture conditions**

The expression of *BCRP* in the *anti-BCRP*-ribozyme-transfectant cell line EPG85-257RNOV-RzB1 (designated *anti-BCRP*-ribozyme transfected cell line) is inhibited by 50% with respect to the *BCRP* overexpressing line EPG85-257RNOV as deduced from the mRNA level (Kowalski, P., Stein, U., Scheffer, G.L., and Lage, H. (2002) *Cancer Gene Ther.* **9**, 579-586). Cells were grown in the absence of cytostatic drugs for 96 hours on 12 well-plates for transport assays. In the control line EPG85-257P, BCRP was below detection level. MDR1 Pgp and MRP1 synthesis proved to be comparable in all sublines except for MDR1 Pgp in the *MDR1* overexpressing cell line EPG85-257RDB (Kowalski, P., Stein, U., Scheffer, G.L., and Lage, H. (2002) *Cancer Gene Ther.* **9**, 579-586). Cell lines were grown in Leibovitz L 15 medium (Bio Whittaker, Grand Island, NY, USA), 1 mM L-glutamine, 6.25 mg/ml fetuin, 80 IE/1 insulin, 2.5 mg/ml transferrin, 1 g/l glucose, 1.1 g/l $NaHCO_3$, 1% minimal essential vitamins, and 20,000 kIE/1 trasylol in a humidified atmosphere of 5% $CO_2$ at 37 °C. Additionally, EPG85-257RDB cells were grown with 2.5 μg/ml daunorubicin and EPG85-257RNOV cells were grown in the presence of 0.2 μg/ml mitoxantrone.

**Transport Assays**

*Measurement of C6-NBD-PS outward translocation.*

To measure redistribution of C6-NBD-PS from the cytoplasmic to the exoplasmic leaflet, labelling of the cytoplasmic leaflet is preconditional. Previously, we have introduced a method to achieve efficient labeling of the plasma membrane inner leaflet of the gastric carcinoma cell line EPG85-257 (Pohl, A., Lage, H., Müller, P., Pomorksi, T., and Herrmann. A. (2002) *Biochem. J.* **365**, 259-268). Briefly, cells were preincubated with or without MDR inhibitors, labelled with 5 μM C6-NBD-PS, and incubated at 20°C for 30 minutes to allow inward movement of the NBD analogue. C6-NBD-PS remaining on the cell surface was extracted twice by incubation with 2% (w/v) BSA in mPBS for 10 minutes on ice. Before starting the outward translocation assay, the medium was removed, and cells were washed with ice-cold mPBS. For t=0', ice-cold medium containing 2% (w/v) BSA and 5 mM DFP ( = diisopropyl fluoro-phosphate) in mPBS with or without inhibitor was immediately added to the cell dish for 10 minutes. To measure C6-NBD-PS outward translocation, medium prewarmed to 37°C was added to the dish, and cells were incubated at 37°C for 30 minutes (t = 30'). Removal of the BSA-containing media and a second extraction with 2% BSA in mPBS for 10 minutes terminated incubations, followed by lipid analysis.

*Measurement of C6-NBD-PC outward translocation*

Translocation of newly synthesized C6-NBD-PC to the exoplasmic leaflet of the plasma membrane was assessed at 15°C as described by van Helvoort et al. (van Helvoort, A., Smith, A., Sprong, H., Fritzsche, I., Schinkel, A., Borst, P., and van Meer, G. (1996) *Cell* **87**, 507-517). Cells were incubated at 15°C with 25 μM C6-NBD-PA in 1% w/v BSA in mPBS with or without inhibitors. After 180 minutes, BSA-containing media were collected, and cells subjected to a 30 minute back exchange incubation on ice with 1% BSA in mPBS, followed by lipid analysis of cells and media.

Lipid analysis

After the incubations, the second BSA back exchange medium was pooled with the first, and cells scraped into mPBS. For analysis of outward translocation and of metabolic conversion of C6-NBD-PS, lipids from both scraped cells and media were extracted with isopropanol/chloroform/(5.5:1, v/v) to prevent substantial loss into the aqueous phase. Samples were centrifuged at 780 g for 5 minutes, the supernatant transferred into new glass tubes and dried, dissolved in chloroform/methanol (1:1) and quantified spectroscopically. For C6-NBD-PC analysis, lipids from both cells and incubation media were extracted by the method of Bligh and Dyer (Bligh, E., and Dyer, W. (1959) *Can. J Biochem. Physiol.* **37**, 911-917) using 20 mM acetic acid in the aqueous phase. After 2-D separation [I, chloroform/methanol/25% ammonium hydroxide (65:25:4, v/v); II, chloroform/acetone/methanol/acetic acid/water (10:4:2:2:1, v/v)] on thin layer chromatography plates, fluorescent lipid spots were visualized under ultraviolet light, scraped, and quantified as described before (Pomorski, T., Müller, P., Zimmermann, B., Burger, K., Devaux, P., and Herrmann, A. (1996) *J. Cell Sci.* **109**, 687-698).

*Annexin assay*

To measure exposure of endogenous PS on the cell surface, cells were incubated on ice for 10 minutes in the dark with 9 nM FITC-Annexin V in binding buffer. Two minutes prior to the end of the incubation, propidium iodide (final 1.9 μM) was added. Cells were then washed three times, detached from the dishes through pipetting, and suspended in binding buffer. Measurement was performed with a FACSCalibur flow cytometer (Becton Dickinson, St. Louis, MO) 5 minutes after the end of incubation. Regions were set to exclude subcellular particles, and only single cells were counted (10 000 cells / sample). A further gate excluded damaged cells with elevated propidium iodide staining. Data were analyzed by Becton Dickinson CellQuest software.

*Miscellaneous*

Results are given as mean +/- standard error (S.E.M.).

## Example 6

Outward translocation of C6-NBD-PC by BCRP in gastric carcinoma cells

C6-NBD-PC outward transport was examined in BCRP overexpressing cells and control cells following incubation with C6-NBD-PA. It has been shown (Pagano, R., Martin, O., Schroit, A., and Struck, D. (1981) *Biochemistry* **20**, 4920-4927) that C6-NBD-PA is partially converted to C6-NBD-diacylglycerol (-DG) which rapidly crosses the plasma membrane and becomes available for intracellular synthesis of C6-NBD-PC and C6-NBD-PE. When transport of the newly synthesized analogues to the cell surface is measured at 15°C, vesicular traffic can be excluded, being is blocked at this temperature (van Genderen, 1., and van Meer, G. (1995) *J. Cell Biol.* **131**, 645-654).

After 180 minutes at 15°C, 44% of C6-NBD-PC were extracted in the BSA medium of the *BCRP* overexpressing subline, while the BSA medium of control cells contained only 24% of C6-NBD-PC (Fig. 7A). To verify that the enhanced outward translocation of C6-NBD-PC was due to overexpression of *BCRP,* the present inventors investigated outward transport in anti-*BCRP*-ribozyme transfected cells. Indeed, they found significantly reduced outward translocation of C6-NBD-PC in these cells (35%) in comparison to *BCRP* overexpressing cells. However, the outward redistribution of the lipid analogue was still higher in anti-*BCRP*-ribozyme transfected cells than in control cells.
In all three sublines, C6-NBD-PE synthesis from the respective PA (phosphatidic acid) precursor, being at the detection level, was too low for determination of specific outward transport.

*Outward translocation of C6-NBD-PS by BCRP in gastric carcinoma cells*

Unlike for C6-NBD-PC, the lipid precursor C6-NBD-PA is not metabolically converted to the respective PS analogue. Therefore, the present inventors applied the approach recently developed by themselves to label the inner plasma membrane leaflet with C6-NBD-PS (Pohl, A., Lage, H., Müller, P., Pomorksi, T., and Herrmann. A. (2002) *Biochem. J.* **365**, 259-268). In that study, they had found rapid C6-NBD-PS inward transport in EPG85-257RDB cells due to the presence of an aminophospholipd translocase activity (for reviews see (Devaux, P. (2000) *Biochimie* **82**, 497-509; Daleke, D.L., and Lyles, J.V. (2000) *Biochim. Biophys. Acta* **1486,** 108-127).

Following this approach, C6-NBD-PS was introduced into the exoplasmic leaflet of the gastric carcinoma cells on ice. Subsequent to intracellular labelling of cells with C6-NBD-PS, its translocation back to the exoplasmic leaflet was determined. C6-NBD-PS and its metabolites appearing on the cell surface were extracted by BSA present in the incubation medium. After a 30 minute incubation at 37°C, extracted lipid analogues were separated from cells and medium, and the respective amount of analogues was quantified by measuring the fluorescence intensity (see above). In *BCRP* overexpressing cells, 46% of C6-NBD-PS was extracted into the medium, significantly more than in control cells (34%) (Fig. 7B). In anti-*BCRP*-ribozyme transfected cells, the outward translocation of the PS analogue (42%) was lower in comparison to *BCRP* overexpressing cells, but still higher with respect to control cells (Fig. 7B). This strongly suggests a BCRP mediated outward movement of phospholipids.

Pretreatment with 10 μM of the BCRP specific inhibitor Tryprostatin A or the multispecific MDR-modulator GF120918 (inhibiting MDR1 P-gp and BCRP) (de Bruin, M., Miyake, K., Litman, T., Robey, R., and Bates, S. (1999) *Cancer Lett.* **146**, 117-126) caused a sharp and highly significant decrease in the amount of C6-NBD-PS in the BSA containing medium of *BCRP* overexpressing cells (Fig. 8), reducing the amount of extracted analogues near to the level found for control cells in the absence of inhibitors. Inhibitors did not affect PS transport significantly in control cells.

A significant part of internalized C6-NBD-PS was metabolized in the human gastric carcinoma cell line EPG85-257 (Pohl, A., Lage, H., Müller, P., Pomorksi, T., and Herrmann. A. (2002) *Biochem. J.* **365**, 259-268). The major metabolic product was C6-NBD-PE which is confirmed by the findings of this application. About 45 % and 55 % of the C6-NBD-PS were converted to C6-NBD-PE in the *BCRP* overexpressing sublines and in the parental cell line, respectively. Remarkably, the present inventors did not observe a BCRP-mediated outward translocation of C6-NBD-PE (data not shown).

Experiments were performed at 15°C (see Outward translocation of C6-NBD-PC). However, outward transport of the PS analogue is reduced at this temperature and no clear difference in transport between control and *BCRP* overexpressing cells could be observed (data not shown).

To reveal whether Tryprostatin A and GF120918 may also inhibit lipid transport activity of MDR1 Pgp, the present inventors measured the outward translocation of C6-NBD-PS in the *MDR1 Pgp* overexpressing subline (EPG85-257RDB) (Fig. 8). While GF120918 caused a significant reduction of PS analogue outward translocation, Tryprostatin A did not affect the MDR1 Pgp lipid transport activity. This supports the present inventors' finding about specific inhibition of BCRP by Tryprostatin A. In contrast, GF120918 proves to inhibit ABC transporters less specifically.

*BCRP overexpressing cells expose more endogenous PS than controls*

Exposure of endogenous PS in *BCRP* overexpressing cells and controls was assessed by flow cytometry. Labelling with both FITC-Annexin V, a high affinity PS binding protein (37), and the membrane-impermeable nucleic acid stain propidium iodide, allows detection of PS present on the outer plasma membrane leaflet of cells with intact cell membranes. Figure 9 shows a typical experiment. The results of several experiments are summarized in figure 10. Since variation between independent experiments was significant (ANOVA, p<0.05), the control (without Tryprostatin A) of each experiment was set to 100%. Binding of Annexin V was clearly enhanced for BCRP-overexpressing cells with respect to control cells. As revealed by ANOVA of original data, the differences were highly significant (p=0.016) and were not depend on the experiment (p=0.638). For *anti-BCRP*-ribozyme transfected cells, binding of Annexin V was intermediate between values of *BCRP* overexpressing and control cells (ANOVA, p=0.181). Preincubation of *BCRP* overexpressing cells with 5 µM Tryprostatin A reduced the level of Annexin V binding to that of control cells without inhibitor. Annexin V binding to control cells was not affected by treatment with Tryprostatin A (data not shown).

Previously, it had been shown that half size transporters are functionally involved in phospholipid and cholesterol trafficking in macrophages (Klucken, J., Büchler, C., Orso, E., Kaminski, W. E., Porsch-Özcürümez, M.., Liebisch, G., Kapinsky, M., Diederich, W., Drobnik, W., Dean, M., Allikmets, R., and Schmitz, G. (2000) Proc. Natl. Acad. Sci USA . 97, 817-822; Schmitz, G., Langmann, T., and Heimer, S. (2001) J. Lip. Res. **42**, 1513-1520). However, while several full size ABC transporters have been shown to mediate translocation of various lipids from the cytoplasmic to exoplasmic leaflet of the plasma membrane, it was not known whether half size ABC proteins may have similar lipid transport activity.

In this application, the present inventors have shown that human half size ABC transporters can be candidate lipid transporters. Using precursor labelling as well as a recently developed assay for labelling of the cytoplasmic leaflet (Pohl, A., Lage, H., Müller, P., Pomorksi, T., and Herrmann. A. (2002) *Biochem. J.* **365**, 259-268), a significantly higher outward movement of the fluorescent lipid analogues, C6-NBD-PC and C6-NBD-PS, was found in *BCRP* overexpressing human gastric carcinoma cells compared to control cells. Transport is highly sensitive to the expression level of *BCRP* and to BCRP inhibitors, strongly supporting the role of this protein in the translocation of lipid analogues across the plasma membrane. Moreover, enhanced binding of FITC-Annexin V to *BCRP* overexpressing cells suggests a role for BCRP in the exposure of endogenous PS to the exoplasmic leaflet.

To study the lipid transport activity of BCRP and its lipid specificity, the present inventors measured the movement of two different phospholipid analogues, C6-NBD-PC and C6-NBD-PS, from the inner to the outer leaflet of the plasma membrane. Incubation of the *BCRP* overexpressing and the control cell line with C6-NBD-PA at 15°C in the presence of BSA enabled us to investigate the translocation of the intracellularly synthesized C6-NBD-PC from the cytoplasmic to the exoplasmic plasma membrane leaflet by continuous incubation of cells in the presence of BSA.

However, C6-NBD-PA is not suitable for biosynthetical conversion into C6-NBD-PS. For this reason, C6-NBD-PS was inserted into the cytoplasmic leaflet of the plasma membrane via the ubiquitous aminophospholipid translocase activity, rapidly transporting PS from the outer to the inner plasma membrane leaflet (see (Pohl, A., Lage, H., Müller, P., Pomorksi, T., and Herrmann. A. (2002) *Biochem. J.* **365**, 259-268)). Subsequently, by continuous incubation of cells in the presence of BSA, C6-NBD-PS outward movement was monitored. Inward transport of the analogue, e.g. via the aminophospholipid translocase, does not interfere with the assay, because BSA rapidly extracts C6-NBD-phospholipid analogues (39).

Compared to controls, a strongly increased outward translocation of C6-NBD-PC and C6-NBD-PS was found *in BCRP* overexpressing cells. The translocation of both analogues was reduced in *anti-BCRP*-ribozyme transfected cells with respect to *BCRP* overexpressing cells, but still higher than in control cells, supporting a role for BCRP in transport of C6-NBD-PC and -PS to the cell surface. Consistent with this, outward transport of the PS analogue was sensitive to BCRP inhibitors Tryprostatin A and GF120918. However, the affinity and/or transport activity of for BCRP may differs between both analogues depending on the temperature. A BCRP mediated outward transport at 15°C was observed for C6-NBD-PC, but not for C6-NBD-PS. Only at higher temperature (37°C) we found a BCRP mediated outward transport for the PS analogue.

Several full size ABC transporters, among them MDR1 Pgp, MDR2/3 Pgp, MRP1 and ABCA1 have been found to mediate lipid transport (9,15-17,19,20). By using specific inhibitors for MRP1 and ABCA1, MK 571 and glyburide, the present inventors had have previously shown that outward translocation of lipid analogues in EPG85-257 cells cannot be ascribed to the activity of MRP1 or ABCA1 (Pohl, A., Lage, H., Müller, P., Pomorksi, T., and Herrmann. A. (2002) *Biochem. J.* **365**, 259-268). Furthermore, the present inventors could rule out in the findings of this application that the well-established lipid transport activity of MDR1 Pgp is responsible for outward directed transport of lipid analogues in *BCRP* overexpressing cells. In summary, the present inventors' findings show that BCRP is able to mediate outward directed transport of lipids, in particular several fluorescent lipid analogues.

In order to study the exposure of endogenous PS at the surface of EPG85-257RNOV cells, binding of fluorescent Annexin V was assessed. In *BCRP* overexpressing cells, binding of this PS probe to the cell surface was significantly higher than for control cells, while binding to *anti-BCRP*-ribozyme transfected cells with a lower expression of *BCRP* was only slightly enhanced with respect to control cells. In the presence of the inhibitor Tryprostatin A, binding of Annexin V to *BCRP* overexpressing cells was reduced to the level found for control cells. Taken together, these results suggest a role of BCRP in translocating endogenous PS to the outer plasma membrane leaflet. Increased exposure of endogenous PS has been previously reported for tumor cells versus non-tumorigenic cells (Utsugi, T., Schroit, A., Connor, J., Bucana, C., and Fidler, I. (1991) *Cancer Res.* **51**, 3062-3066) and recently for *MDR1* overexpressing EPG85-257RDB cells versus controls (Pohl, A., Lage, H., Müller, P., Pomorksi, T., and Herrmann. A. (2002) *Biochem. J.* **365,** 259-268).

Different to the bacterial half-size ABC transporter LmrA of *Lactococcus lactis,* which mediates transbilayer movement of C6-NBD-PE but not of C6-NBD-PC (41), the present inventors did not observe a BCRP-dependent outward movement of C6-NBD-PE which has been formed intracellularly as a metabolic product of C6-NBD-PS. This clearly suggests differences in the substrate specificity among half-size transporter. Nevertheless, the enhanced outward movement of two different phospholipid analogues, C6-NBD-PC and C6-NBD-PS, in *BCRP* overexpressing cells indicates that the BCRP mediated lipid transport might be of relatively broad lipid specificity. The latter is supported by observations of Litman et al. (Raggers, R. J., Pomorski, T., Holthuis, J. C. M., Kalin, N., and van Meer, G. (2000) *Traffic* **1**, 226-234) suggesting that a fluorescent analogue of ceramide (BODIPY C5-ceramide) might be a substrate of BCRP. This suggestion was based on the intracellular distribution of the analogue in a breast cancer cell line affected by BCRP. However, one may wonder whether the specific chemical nature of the analogues is responsible for the observed transport. Although short-chain NBD lipids have provided important information on the cellular dynamics of the respective endogenous lipids (Devaux, P.F., Fellmann, P., and Hervé, P. (2002) *Chem. Phys. Lip.* **116**, 115-134), they reflect the behavior of endogenous lipids only to a particular level, limited by the presence of the short fatty acid chain and a bulky reporter group. Nevertheless, the enhanced exposure of endogenous PS in *BCRP* overexpressing cells provides an indication that endogenous phospholipids are recognized as substrates for BCRP as well. Broader specificity towards lipid transport substrates may not be surprising, and has been documented for full the size ABC transporter MDR1 Pgp. MDR1 Pgp mediated translocation has been demonstrated for short-chain analogues of PC, PE, for glucosylceramide without an NBD group (van Helvoort, A., Smith, A., Sprong, H., Fritzsche, I., Schinkel, A., Borst, P., and van Meer, G. (1996) *Cell* **87**, 507-517), for the endogenous 1-O-alkyl-2-acetyl-sn-glycero-3-phosphocholine, PAF (Ernest, S., and Bello-Reuss, E. (1999) *J. Am. Soc. Nephrol.* **10**, 2306-2313), and for endogenous PS as well as its analogues (Pohl, A., Lage, H., Müller, P., Pomorksi, T., and Herrmann. A. (2002) *Biochem. J.* **365**, 259-268).

Further studies are warranted on whether the lipid transport activity of BCRP is comparable to that of MDR1 Pgp. Without wishing to be bound by any theory, the present inventors believe MDR1 Pgp to act as a (drug) floppase, transporting amphiphilic molecules integrated into the plasma membrane either from the cytoplasmic to the exoplasmic membrane leaflet, or directly into the extracellular medium, lowering the intracellular drug concentration (Higgins, C., and Gottesman, M. (1992) *TIBS* **17**, 18-21). In particular, it has to be elucidated whether BCRP forms homodimers, or rather heterodimers with other ABCG family members, to mediate lipid translocation. The idea of BCRP functioning as a homodimer has recently been supported by studies in which this multidrug transporter was functionally characterized in a heterologous system (Sf9 insect cells) (Özvegy, C., Litman, T., Szakács, G., Nagy, Z., Bates, S., Váradi, A., and Sarkadi, B. (2001) *Biochem. Biophys. Res. Comm.* **285**, 111-117; 45. Kage, K., Tsukahara, S., Sugiyama, T., Asada, S., Ishikawa, E., Tsuoro, T., and Sugimoto, Y. (2002) *Int. J Cancer.* **97**, 626-630).

The features of the present invention disclosed in the specification and tables, the claims and/or in the accompanying drawings, may, both separately, and in any combination thereof, be material for realising the invention in various forms thereof.

Table 1: $IC_{50}$ values for mitoxantrone and daunorubicin with or without TPS-A at a concentration of 1, 5 or 10 µM

| | without TPS-A | | + 1 µM TPS-A | | + 5 µM TPS-A | | + 10 µM TPS-A | |
|---|---|---|---|---|---|---|---|---|
| | $IC_{50}$ [µg/ml] | RR[1] | $IC_{50}$ [µg/ml] | DMF[2] | $IC_{50}$ [µg/ml] | DMF[2] | $IC_{50}$ [µg/ml] | DMF[2] |
| *Mitoxantrone* | | | | | | | | |
| MCF-7 | 0.007 | 1.0 | 0.011 | 0.6 | 0.008 | 0.9 | 0.005 | 1.4 |
| MCF-7/pcDNA3 | 0.01 | 1.4 | 0.012 | 0.8 | 0.0018 | 5.5 | 0.0015 | 6.6 |
| MCF-7/BCRP clone8 | 0.12 | 17.1 | 0.112 | 1.1 | 0.031 | 3.9 | 0.0019 | 63.2 |
| MCF-7/AdrVp | 48 | 6857 | 22 | 2.2 | 13 | 3.7 | 0.8 | 60.0 |
| EPG85-257P | 0.013 | 1.0 | 0.006 | 2.2 | 0.001 | 13.0 | 0.0001 | 130.0 |
| EPG85-257RNOV | 3.8 | 292.3 | 0.78 | 4.9 | 0.55 | 6.9 | 0.15 | 25.3 |
| EPG85-257RDB | 0.6 | 46.1 | 0.72 | 0.8 | 0.85 | 0.7 | 0.92 | 0.7 |
| *Daunorubicin* | | | | | | | | |
| MCF-7 | 0.018 | 1.0 | 0.016 | 1.1 | 0.0097 | 1.9 | 0.0125 | 1.4 |
| MCF-7/pcDNA3 | 0.019 | 1.1 | 0.014 | 1.4 | 0.008 | 2.4 | 0.0038 | 5.0 |
| MCF-7/BCRP clone8 | 0.047 | 2.6 | 0.046 | 1.0 | 0.023 | 2.0 | 0.011 | 4.3 |
| MCF-7/AdrVp | 1.45 | 80.5 | 1.4 | 1.0 | 1.5 | 0.8 | 0.37 | 3.9 |
| EPG85-257P | 0.0074 | 1.0 | 0.0078 | 0.9 | 0.0066 | 1.1 | 0.0078 | 0.9 |
| EPG85-257RNOV | 0.036 | 4.9 | 0.03 | 1.2 | 0.011 | 3.3 | 0.011 | 3.3 |
| EPG85-257RDB | 3.15 | 425.7 | 2.9 | 1.1 | 3.3 | 1.0 | 3.2 | 1.0 |

[1]  RR, relative resistance

[2]  DMF, dose modifying factor, defined as the $IC_{50}$ for the antitumor drug without TPS-A divided by the $IC_{50}$ with TPS-A

**Table 2: IC$_{50}$ values for mitoxantrone and daunorubicin with or without GF120918 at a concentration of 0.5 µM or 1.0 µM**

| | without GF120918 | | + 0.5 µM GF120918 | | + 1.0 µM GF120918 | |
|---|---|---|---|---|---|---|
| | IC$_{50}$ [µg/ml] | RR[1] | IC$_{50}$ [µg/ml] | DMF[2] | IC$_{50}$ [µg/ml] | DMF[2] |
| *Mitoxantrone* | | | | | | |
| MCF-7 | 0.007 | 1.0 | 0.017 | 0.4 | 0.018 | 0.4 |
| MCF-7/pcDNA3 | 0.01 | 1.4 | 0.0053 | 1.9 | 0.0037 | 2.7 |
| MCF-7/BCRP clone8 | 0.12 | 17.1 | 0.048 | 2.5 | 0.014 | 8.6 |
| MCF-7/AdrVp | 48 | 6857 | 18 | 2.7 | 1.5 | 32 |
| EPG85-257P | 0.013 | 1.0 | 0.0015 | 8.7 | 0.0014 | 9.2 |
| EPG85-257RNOV | 3.8 | 292.3 | 0.1 | 38.0 | 0.0828 | 45.9 |
| EPG85-257RDB | 0.6 | 46.1 | 0.0048 | 125.0 | 0.0052 | 115.4 |
| *Daunorubicin* | | | | | | |
| MCF-7 | 0.018 | 1.0 | 0.019 | 0.9 | 0.0185 | 1.0 |
| MCF-7/pcDNA3 | 0.019 | 1.1 | 0.016 | 1.2 | 0.015 | 1.3 |
| MCF-7/BCRP clone8 | 0.047 | 2.6 | 0.036 | 1.3 | 0.0145 | 3.2 |
| MCF-7/AdrVp | 1.45 | 80.5 | 0.95 | 1.5 | 0.67 | 2.2 |
| EPG85-257P | 0.0074 | 1.0 | 0.008 | 0.9 | 0.0078 | 0.9 |
| EPG85-257RNOV | 0.036 | 4.9 | 0.0175 | 2.1 | 0.012 | 3.0 |
| EPG85-257RDB | 3.15 | 425.7 | 0.016 | 196.9 | 0.012 | 262.5 |

[1]    RR, relative resistance

[2]    DMF, dose modifying factor, defined as the IC$_{50}$ for the antitumor drug without GF120918 divided by the IC$_{50}$ with GF120918

**Claims**

1.  Use of tryprostatin A or any protonated form thereof for the manufacture of a medicament for the treatment of a cancerous disease, wherein a reversal of drug resistance in cancer cells is desired.

2.  Use according to claim 1, wherein the drug resistance is a multidrug resistance.

3.  Use according to any of the foregoing claims, wherein a dose modifying factor (DMF) is $\geq 1$, the DMF being defined by the formula

$$\text{DMF} = \frac{\text{IC}_{50} \text{ (in the absence of tryprostatin A)}}{\text{IC}_{50} \text{ (in the presence of tryprostatin A)}}$$

    $\text{IC}_{50}$ being the concentration of an anti-cancer pharmaceutical at which said anti-cancer pharmaceutical achieves half-maximum inhibition of growth of cancer cells.

4.  Use according to claim 3, wherein the DMF $\geq 2$.

5.  Use according to claim 4, wherein the DMF $\geq 6$.

6.  Use according to claim 5, wherein the DMF $\geq 60$.

7.  Use according to claim 6, wherein the concentration of tryprostatin A is $\geq 5 \, \mu\text{M}$.

8.  Use according to any of the foregoing claims, wherein the drug resistance is a resistance against a compound/combination of compounds selected from the group comprising mitoxantrone, camptothecin-analogues, in particular topotecan, doxorubicin, bisantrene, vinblastin, paclitaxel and colchicin.

9.  Use according to any of the foregoing claims, wherein the drug resistance is a non P-gp-mediated/non MRP drug resistance.

10. Use according to any of the foregoing claims, wherein the drug resistance is a BCRP-mediated drug resistance.

11. Use according to any of the foregoing claims, wherein the cancerous disease is selected form the group comprising breast, gastric, brain, prostate, liver, colon, ovarian, testicular, small intestinal cancer, non-Hodgkin's lymphoma, and leukemia.

12. Use according to any of the foregoing claims, wherein an increase of drug accumulation of $\geq 40\%$ in the cancer cells is desired, said increase of drug accumulation being a ratio of drug accumulation measured in the presence and absence of tryprostatin A.

13. Use according to any of the foregoing claims, wherein tryprostatin A is administered concomitantly with an anti-cancer pharmaceutical.

14. Use according to claim 13, wherein the anti-cancer pharmaceutical is selected from the group comprising mitoxantrone, camptothecin-analogues, in particular topotecan, doxorubicin, bisantrene, vinblastin, paclitaxel and colchicin.

15. Use according to any of the foregoing claims, wherein tryprostatin A is used in a concentration in the range of from $1 \, \mu\text{M}$ - $100 \, \mu\text{M}$.

16. Use according to claim 15, wherein tryprostatin A is used in a concentration in the range of from $1 \, \mu\text{M}$ - $50 \, \mu\text{M}$.

17. Use according to any of the foregoing claims, wherein the cancerous disease is in a human.

18. Use of tryprostatin A or any protonated form thereof for the identification of compounds that reverse a BCRP-mediated drug resistance.

19. Use of tryprostatin (A) or any protonated form thereof for the manufacture of a medicament for treating diseases associated with lipid transport in and out of cells.

20. Use according to claim 19, wherein the lipid transport is BCRP-mediated.

21. Use according to any of claims 19-20, wherein an inhibition of such lipid transport is desired.

22. A method of screening for compounds capable of chemosensitizing multidrug-resistant cells comprising the steps:

- Administering a subject compound to be tested to a non P-GP/non MRP multiple drug resistant cell,
- administering an anti-cancer pharmaceutical to which the cell is resistant,
- measuring a response, and
- comparing the response with a response achieved by administering tryprostatin A to a non P-gp/non MRP multiple drug resistant cancer cell of the same type, that has not been treated with the subject compound to be tested.

23. Compound identified by the method according to claim 22, having a DMF $\geq 2$, said DMF being defined by the formula

$$DMF = \frac{IC_{50} \text{ (in the absence of tryprostatin)}}{IC_{50} \text{ (in the presence of tryprostatin)}} ,$$

$IC_{50}$ being the concentration of an anti-cancer pharmaceutical at which said anti-cancer pharmaceutical achieves half-maximum inhibition of growth of cancer cells.
and said compound causing an increase of drug accumulation of $\geq 40\%$, wherein said increase of drug accumulation is the ratio of drug accumulation measured in the presence and absence of tryprostatin A.

**Figure 1**

Figure 2

**Figure 3**

Figure 4

**Figure 5**

**Figure 6**

**Figure 7**

**Figure 8**

Figure 9

**Figure 10**

**European Patent**
**Office**

**PARTIAL EUROPEAN SEARCH REPORT**

which under Rule 45 of the European Patent Convention
shall be considered, for the purposes of subsequent
proceedings, as the European search report

Application Number

EP 03 00 7683

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | PATENT ABSTRACTS OF JAPAN vol. 1997, no. 07, 31 July 1997 (1997-07-31) & JP 09 059275 A (RIKAGAKU KENKYUSHO), 4 March 1997 (1997-03-04) * abstract * --- | 1-23 | A61K31/4985 A61P43/00 G01N33/50 |
| Y | KONDOH MASUO ET AL: "Effects of tryprostatin derivatives on microtubule assembly in vitro and in situ." JOURNAL OF ANTIBIOTICS (TOKYO), vol. 51, no. 8, August 1998 (1998-08), pages 801-804, XP009013623 ISSN: 0021-8820 * page 801 * * page 803 * --- -/-- | 1-23 | |

TECHNICAL FIELDS
SEARCHED     (Int.Cl.7)

A61K
A61P
G01N

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 28 July 2003 | Domingues, H |

EPO FORM 1503 03.82 (P04C07)

| | | | |
|---|---|---|---|
| **European Patent**<br>**Office** | **INCOMPLETE SEARCH**<br>**SHEET C** | Application Number<br>EP 03 00 7683 |

Claim(s) searched incompletely:
      19-21

Claim(s) not searched:
      3-7, 23

Reason for the limitation of the search:

Present claims 2-7 and 23 refer to the following parameter: dose modifying factor (DMF).
Since it is impossible to compare this parameter with the prior art, the claims lack clarity (Art. 84 EPC) to such an extent that a meaningful search is impossible.

Furthermore, present claims 19-21 relate to diseases defined by reference to a biological mechanism that may be involved in a wide range of pathological conditions. Support within the meaning of Article 84 EPC and/or disclosure within the meaning of Article 83 EPC is to be found, however, for only a very limited number of diseases. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the treatment of cancer (pg. 8 and claim 11).

EP 1 464 336 A1

**European Patent Office**

**PARTIAL EUROPEAN SEARCH REPORT**

Application Number

EP 03 00 7683

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | USUI TAKEO ET AL: "Tryprostatin A, a specific and novel inhibitor of microtubule assembly." BIOCHEMICAL JOURNAL, vol. 333, no. 3, 1 August 1998 (1998-08-01), pages 543-548, XP002247384 ISSN: 0264-6021 * page 564 - page 547 * | 1-23 | |
| Y | ZHAO SHUO ET AL: "Biological activity of the tryprostatins and their diastereomers on human carcinoma cell lines." JOURNAL OF MEDICINAL CHEMISTRY, vol. 45, no. 8, 11 April 2002 (2002-04-11), pages 1559-1562, XP002247385 April 11, 2002 ISSN: 0022-2623 * the whole document * | 1-23 | TECHNICAL FIELDS SEARCHED (Int.Cl.7) |
| Y | HE H ET AL: "FUMITREMORGIN C ANALOGS THAT REVERSE MITOXANTRONE RESISTANCE IN HUMAN COLON CARCINOMA CELLS" MEDICINAL CHEMISTRY RESEARCH, BIRKHAEUSER, BOSTON, US, vol. 9, no. 6, 1999, pages 424-437, XP001073664 ISSN: 1054-2523 * page 424 - page 425 * * page 430 * | 1-23 | |

-/--

EPO FORM 1503 03.82 (P04C10)

**EP 1 464 336 A1**

| | European Patent Office | PARTIAL EUROPEAN SEARCH REPORT | Application Number |
|---|---|---|---|
| | | | EP 03 00 7683 |

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | ALLEN JOHN D ET AL: "Potent and specific inhibition of the breast cancer resistance protein multidrug transporter in vitro and in mouse intestine by a novel analogue of fumitremorgin C." MOLECULAR CANCER THERAPEUTICS, vol. 1, no. 6, April 2002 (2002-04), pages 417-425, XP002247386 April, 2002 ISSN: 1535-7163 * page 417 * * page 423 - page 424 * | 1-23 | |
| Y | VAN LOEVEZIJN ARNOLD ET AL: "Inhibition of BCRP-mediated drug efflux by fumitremorgin-type indolyl diketopiperazines." BIOORGANIC & MEDICINAL CHEMISTRY LETTERS, vol. 11, no. 1, 2001, pages 29-32, XP002247387 ISSN: 0960-894X * the whole document * | 1-23 | **TECHNICAL FIELDS SEARCHED** (Int.Cl.7) |
| Y | US 2002/169111 A1 (HE HAIYIN ET AL) 14 November 2002 (2002-11-14) * claims 1-63 * | 1-23 | |
| A | SIKIC B I: "Modulation of multidrug resistance: a paradigm for translational clinical research." ONCOLOGY (WILLISTON PARK, N.Y.) UNITED STATES MAY 1999, vol. 13, no. 5A, May 1999 (1999-05), pages 183-187, XP009013593 ISSN: 0890-9091 * the whole document * | 1-23 | |

EPO FORM 1503 03.82 (P04C10)

31

**ANNEX TO THE EUROPEAN SEARCH REPORT
ON EUROPEAN PATENT APPLICATION NO.**

EP 03 00 7683

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2003

| Patent document cited in search report | | | Publication date | Patent family member(s) | | Publication date |
|---|---|---|---|---|---|---|
| JP 09059275 | A | | 04-03-1997 | NONE | | |
| US 2002169111 | A1 | | 14-11-2002 | US 6372775 B1 | | 16-04-2002 |
| | | | | US 2003083230 A1 | | 01-05-2003 |
| | | | | US 2002156015 A1 | | 24-10-2002 |
| | | | | US 6537964 B1 | | 25-03-2003 |

EPO FORM P0459

For more details about this annex : see Official Journal of the European Patent Office, No. 12/82